# EUROPÄISCHE PATENTSCHRIFT

(11) **EP 2 615 082 B1**
(45) Veröffentlichungstag und Bekanntmachung des Hinweises auf die Patenterteilung: **06.04.2016**
(21) Anmeldenummer: 12006015.7
(22) Anmeldetag: 23.08.2012
(51) Int. Cl.: C07C 269/02, C07C 273/18, C07C 209/56, C07C 213/02, C07C 211/45, C07C 211/47, C07C 211/46, C07C 217/84, C07C 271/28, C07C 271/12, C07C 271/44, C07C 271/20, C07C 275/20

(54) **Herstellung von Carbamaten über eine basenkatalysierte Lossen- Umlagerung**
Production of carbamates by means of base-catalyzed Lossen rearrangement
Fabrication de carbamates au moyen d'un réarrangement de Lossen à catalyse basique

(30) Priorität: 10.01.2012 DE 102012100127
(43) Veröffentlichungstag der Anmeldung: 17.07.2013
(73) Patentinhaber: Karlsruher Institut für Technologie, 76131 Karlsruhe (DE)
(72) Erfinder: Kreye, Oliver, 75203 Königsbach (DE); Meier, Michael, 76751 Jockgrim (DE)

(56) Entgegenhaltungen:
- EP-A1- 0 190 466
- EP-B1- 1 904 433
- CN-A- 101 219 972
- JP-A- H0 789 926
- US-A- 3 465 024
- HATICE MUTLU ET AL: "TBD catalysis with dimethyl carbonate: a fruitful and sustainable alliance", GREEN CHEMISTRY, Bd. 14, Nr. 6, 16. April 2012 (2012-04-16) , Seite 1728, XP055177987, ISSN: 1463-9262, DOI: 10.1039/c2gc35191a
- PASCAL DUBÉ ET AL: "Carbonyldiimidazole-Mediated Lossen Rearrangement", ORGANIC LETTERS, Bd. 11, Nr. 24, 17. Dezember 2009 (2009-12-17), Seiten 5622-5625, XP055177963, ISSN: 1523-7060, DOI: 10.1021/ol9023387

## Beschreibung

Die vorliegende Erfindung betrifft ein Verfahren zur Herstellung von Carbamaten, Harnstoffderivaten oder aromatischen Aminen mittels einer basenkatalysierten Lossen-Umlagerung.

Carbamate sind eine Stoffgruppe von industrieller Bedeutung, z.B. bei der Herstellung von Insektiziden, Fungiziden und Herbiziden. Weiterhin finden Carbamate Anwendung in der Entwicklung von Pharmazeutika (z. B. Schlafmittel) und in der Organischen Synthesechemie als Schutzgruppen von Aminen (z. B. in der Synthese von Peptiden). Auch als Zwischenprodukte in der organischen Synthese, haben Carbamate eine große Bedeutung, beispielsweise in der pflanzenölverarbeiteten Industrie (Oleochemie).

Konventionell können Carbonsäurederivate über den Hofmann-Abbau oder die Curtius-Umlagerung in die entsprechenden Isocyanate überführt werden, bzw. über eine nachgelagerte Additionsreaktion in Carbamate. Auch die Schmidt-Reaktion erlaubt eine Derivatisierung von Carbonsäurederivaten über einen Umlagerungsschritt zum Isocyanat. Das Isocyanat wird bei der Schmidt-Reaktion durch die Anwesenheit von Wasser unter Abspaltung von CO₂ zum Amin hydrolysiert.

Bei diesen Umlagerungsmethoden ist die industrielle Bedeutung durch mehrere Faktoren stark eingeschränkt. Bei der Curtius-Umlagerung werden Carbonsäureazide als Intermediate erhalten, die eine hohe Toxizität aufweisen und zudem noch stark explosiv sind. Aufgrund dieser Eigenschaften, ist eine großtechnische Nutzung nur unter größten Sicherheitsvorkehrungen durchführbar.Bei der klassischen Hofmann-Umlagerung werden Carbonsäureamide mit alkalischer Bromlösung behandelt, um die Umlagerung einzuleiten. Die Verwendung von Überschüssen des sehr giftigen Broms und Natriumhydroxids machen die Durchführung zu einem stark umweltbelastenden Verfahren, in dem des Weiteren viele Abfallprodukte anfallen. Modernere Varianten der Hofmann-Umlagerung setzen das teure (Bis(trifluoroacetoxy)iodo) benzol oder N-Bromsuccinimid (NBS) ein, welche die Methode allerdings auch nicht ökonomischer gestalten.Die Schmidt-Reaktion erfordert den Einsatz von Stickstoffwasserstoffsäure unter Zusatz einer starken Mineralsäure.

Die Lossen-Umlagerung hat bislang wenig technische Relevanz für die Darstellung von Carbamaten. Bei der klassischen Lossen-Umlagerung werden aktivierte Hydroxamsäurederivate in Isocyanate überführt, wenn sie mit Basen und wasserentziehenden Substanzen erhitzt werden, wie in **Schema 1** verdeutlicht ist. Die intermediär gebildeten Isocyanate können mit Alkoholen eine Additionsreaktion zu den entsprechenden Carbamaten eingehen oder, wie im Schema gezeigt, in Anwesenheit von Wasser zum Amin decarboxylieren.

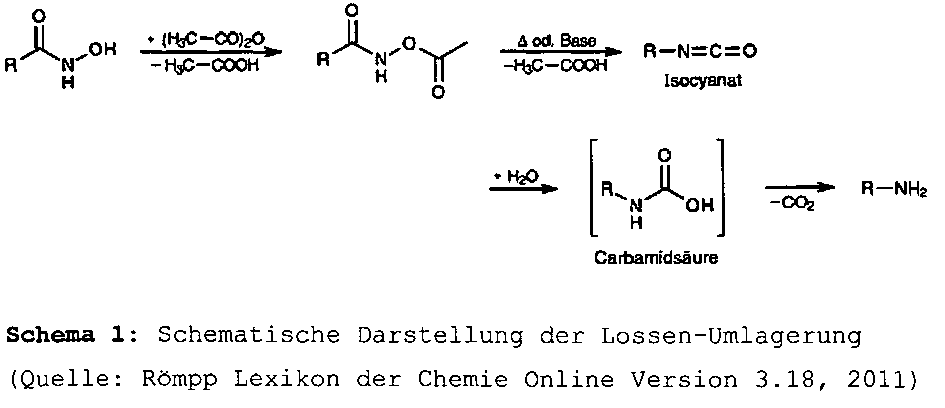

US 3,465,024 offenbart die Darstellung von Isocyanaten ausgehend von acetylierten Hydroxamsäurederivaten über eine basenfreie Lossen-Umlagerung zu Isocyanaten. Die Weiterreaktion zu Carbamaten in einem in situ-Prozess wird nicht thematisiert.

In EP 1904433 B1 wird ein Verfahren zur Herstellung von Carbamat-geschützten oder ungeschützten Aminen beansprucht. Ausgehend von Hydroxamsäuren werden in Gegenwart von mindestens stöchiometrischen Mengen eines Alkohols und mindestens drittelstöchiometrischen Mengen an Alkylphosphonsäureanhydriden, insbesondere T3P (2,4,6-Tripropyl-1,3,5,2,4,6-Trioxatriphosphorinan-2,4,6 trioxid), Carbamate hergestellt. Die Verwendung von Basen, insbesondere Amine, ist in diesem Verfahren optional. Sofern Basen eingesetzt werden, werden sie in mindestens stöchiometrischer Menge zugesetzt. Nachteil dieses Verfahrens ist der Einsatz problematischer Edukte (T3P), die auch nicht wiederverwendet werden können. Sie müssen kostenintensiv entsorgt werden, was vor allem bei phosphorhaltigen Verbindungen problematisch ist. Die tertiären Amine werden hier nicht katalytisch eingesetzt.

Dubé et al. (P. Dubé, N. Nathel, M. Vetelino, M. Couturier, C. Larrivée Aboussafy, S. Pichette, M. L. Jorgensen, M. Hardink, ORGANIC LETTERS, 2009, Vol. 11, No. 24, 5622-5625) haben ein Verfahren der Carbonyldiimidazol (CDI)-vermittelten Lossen-Umlagerung ausgehend von Hydroxamsäuren vorgeschlagen. Hierbei entsteht Isocyanat als Zwischenstufe, welches in Anwesenheit von Benzylalkohol zum entsprechenden Carbamat umgesetzt wird. Nachteile bei diesem Verfahren sind insbesondere der stöchiometrische Verbrauch an CDI und der daraus anfallenden Anteil an Imidazol als Nebenprodukt.

Die bisherigen Verfahren zur Darstellung von Carbamaten sind häufig langwierige Prozesse, die oft sehr viele Reaktionsschritte beinhalten. Auch die Verwendung von teilweise teuren oder auch chemisch aggressiven Reagenzien machen diese Reaktionen unwirtschaftlich. Insbesondere ist der Anfall von Nebenprodukten erheblich, da stöchiometrische Mengen an Hilfsstoffen verbraucht werden.

Es wäre also wünschenswert, wenn Carbamate auf einem einfachen Weg zugänglich wären, ohne dass hierbei bedenkliche Neben- oder Abfallprodukte in erheblichem Maße anfallen. Idealerweise sollte diese Reaktion auf katalytischem Wege erfolgen, sodass die Nebenprodukte minimiert werden.

Ausgehend hiervon ist es Aufgabe der Erfindung, ein Verfahren bereitzustellen, das die Nachteile des Stands der Technik überwindet. Insbesondere ist es Aufgabe der Erfindung, ein Verfahren vorzuschlagen, mit welchem ausgehend von Hydroxamsäuren Carbamate, Harnstoffe und/oder aromatische Amine in einem Eintopfprozess unter katalytischen Bedingungen dargestellt werden können.

Die vorliegende Erfindung löst die Aufgabe durch das in Anspruch 1 beanspruchte Verfahren. Bevorzugte Ausführungsformen des Verfahrens sind in den rückbezogenen Ansprüchen beschrieben.

Die vorliegende Erfindung stellt ein Syntheseverfahren zur Herstellung von Carbamaten oder Harnstoffen der allgemeinen Formel R¹-NH-CO-Nu bereit. Nu ist ein Alkoxyrest der Formel OR² oder ein Alkylamin mit einer Formel NHR¹, wobei R¹ ein ggf. substituierter, linearer, verzweigter oder cyclischer C₁-C₅₀, vorzugsweise C₁-C₂₀, Alkyl-, Alkenyl- oder Allylrest ist, und R² ein ggf. substituierter, linearer, verzweigter oder cyclischer C₁-C₅₀, vorzugsweise C₁-C₂₀, Alkyl-, Alkenyl- oder Allylrest oder ein C₆-C₅₀, vorzugsweise C₆-C₂₀, Aryl- oder C₄-C₅₀, vorzugsweise C₄-C₂₀, Heteroarylrest ist. Besonders bevorzugt ist R² eine Methylgruppe.

Werden bei diesem Verfahren aromatische Hydroxamsäuren der allgemeinen Formel R³-CO-NH-OH eingesetzt, wobei R³ ein C₆-C₅₀, vorzugsweise C₆-C₂₀, Arylrest oder C₄-C₅₀, vorzugsweise C₁-C₂₀, Heteroarylrest ist, so entstehen aromatische Amine der allgemeinen Formel R³-NH₂. Dementsprechend umfasst die vorliegende Erfindung auch ein Verfahren zur Herstellung von aromatischen Aminen der allgemeinen Formel R³-NH₂, welches dieselben Verfahrensschritte umfasst. Die eingesetzten Heteroaryle umfassen vorzugsweise Thiophene, Furane oder Pyridine.

Im Zusammenhang mit der vorliegenden Erfindung bedeutet der Begriff "substituiert" eine Gruppe, in der ein oder mehrere Wasserstoffatome unabhängig mit der gleichen oder verschiedenen Substituenten ersetzt werden. Typische Substituenten umfassen funktionelle Gruppen aus Heteroatomen, wie Sauerstoff, Schwefel, Stickstoff, Halogenen oder Phosphor. Bevorzugte Substituenten sind Halogene (F, Cl, Br, J) bzw. Pseudohalogene wie Fulminate, Thiofulminate, Cyanate, Thiocyanate, Azide, Isocyanate, Isothiocyanate, Isocyanide, Cyanide, oder funktionelle Gruppen wie Amine, Nitrate, Nitrite, Nitrosoverbindungen, Hydroxide, Thiole, Alkoxide, Sulfide, Sulfoxide, Ketone, Thioketone, Aldehyde, Carbonsäuren, Carbonsäureester, - thioester und -amide, Sulfensäuren, Sulfinsäuren, Sulfoxide, Sulfene, Sulfonsäuren, Sulfone, Sulfoximide, Hydrazine, Hydrazone, Azoverbindungen, Phosphaalkine, Phosphane, Phosphine, Phosphaalkane, Phosphorylide, Phosphanoxide, Phosphinsäuren oder Phosphonsäuren.

Das Verfahren umfasst zwei Verfahrensschritte. In einem ersten Schritt wird als Edukt eine Hydroxamsäure der allgemeinen Formel R¹-CO-NH-OH oder R³-CO-NH-OH bereitgestellt. Im zweiten Schritt wird die Hydroxamsäure mit organischen Carbonaten der Formel R²O-CO-OR² in Gegenwart einer katalytischen Menge eines tertiären Amins umgesetzt. R¹, R² und R³ sind definiert wie oben. Das tertiäre Amin hat bei dieser Reaktion als Base eine katalytische Wirkung und wird bei der erfindungsgemäßen Synthese nicht mitumgesetzt, was vorteilhaft hinsichtlich der Vermeidung von Abfallstoffen ist.

Bei Vorliegen von substituierten Resten R¹, R², R³, welche nukleophile funktionelle Gruppen tragen, besteht die Möglichkeit, dass diese Gruppen in einer Nebenreaktion das Carbonat nukleophil angreifen und somit derivatisiert werden können. In den Produkten können diese funktionellen Gruppen somit mit einer Schutzgruppe versehen werden. Dem Fachmann sind solche Gruppen geläufig.

Vorzugsweise wird das organische Carbonat R²O-CO-OR² in einer Menge von 0,1 bis 500 Moläquivalenten, besonders bevorzugt von 2 bis 30 Moläquivalenten, bezogen auf die Menge an Hydroxamsäure eingesetzt.

Das tertiäre Amin wird vorzugsweise in einer Menge von 0,0001 bis 2 Moläquivalenten bezogen auf die molare Menge an Hydroxamsäure zum Reaktionsgemisch hinzugefügt. Besonders bevorzugt werden 0,01 bis 0,3 Moläquivalente eingesetzt. Grundsätzlich wurde festgestellt, dass die Menge an tertiärem Amin die Reaktionsgeschwindigkeit beeinflusst. Geringe Mengen ab 0,0001 Moläquivalenten an tertiärem Amin haben bereits eine katalytische Wirkung, die Reaktionsgeschwindigkeit wird aber vorteilhaft verkürzt, wenn größere Mengen eingesetzt werden.

In einer weiteren bevorzugten Ausführungsform des Verfahrens wird das tertiäre Amin aus der Gruppe 1,5,7-Triazabicyclo-[4.4.0]dec-5-en (TBD), Triethylamin, 1,8-Diazabicyclo[5.4.0] undec-7-en (DBU) und 1,4-Diazabicyclo[2.2.2]octan (DABCO) ausgewählt.

Hydroxamsäuren als Edukte sind relativ einfach zu erhalten durch aus dem Stand der Technik bekannte Methoden, wie beispielhaft in US 2,397,508 offenbart. Hier wird eine Synthese beschrieben, wie man ausgehend von Carbonsäuren bzw. Carbonsäureestern durch Reaktion mit Hydroxylammoniumchlorid Hydroxamsäuren in guten Ausbeuten (>80%) erhält.

In einer bevorzugten Ausführung des erfindungsgemäßen Verfahrens wird der zweite Schritt in Gegenwart eines Alkohols der Formel R²OH durchgeführt, wobei der Rest R² definiert ist wie oben.

Die Anwesenheit des Alkohols R²OH steuert die Produktbildung bei der Verwendung von Hydroxamsäuren mit nicht-aromatischen Resten R¹ in Richtung Carbamat der Formel R¹-NH-CO-OR². In Abwesenheit des Alkohols wird hauptsächlich das Harnstoffderivat der Formel R¹-NH-CO-NHR¹ gebildet. In Anwesenheit des Alkohols R²OH fällt als weiteres Nebenprodukt der Carbonsäureester R¹-CO-OR² an, der aber für die Hydroxamsäuresynthese (s.o.) wieder eingesetzt werden kann. Bei der erfindungsgemäßen basenkatalysierten Lossen-Umlagerung ist also die Produktart und - ausbeute durch die Menge an hinzugefügtem Alkohol orientierbar. In Anwesenheit geringer Mengen des Alkohols von 1 bis 10 Moläquivalenten, besonders bevorzugt 1 bis 3 Moläquivalenten ist die Ausbeute an Carbamat (70-80%) höher, es entstehen weniger Harnstoff (10-15%). Wird die Reaktion in Anwesenheit größerer Mengen an Alkohol R²OH, also mehr als 10 Moläquivalente, durchgeführt, so steigert sich auch die Ausbeute an Carbonsäureester R¹-CO-OR², während die Ausbeute an Carbamat sinkt und Harnstoff nur in Spuren gebildet wird.

In einer besonders bevorzugten Ausführung des Verfahrens werden aliphatische Hydroxamsäuren R¹-CO-NH-OH, Carbonat R²O-CO-OR² und Alkohol R²OH in den Mengenverhältnissen 1:20:2 eingesetzt.

Bei der Verwendung aromatischer oder heteroaromatischer Hydroxamsäuren der Formel R³-CO-NH-OH zur Darstellung von Aminen der Formel R³-NH₂ wirkt sich die Anwesenheit des Alkohols R²OH ebenfalls vorteilhaft für die Reaktionsgeschwindigkeit aus. Die Darstellung dieser aromatischen Amine ist aber grundsätzlich auch in Abwesenheit des Alkohols möglich.

Um gute Ausbeuten an Carbamaten der Formel R¹-NH-CO-OR² oder Aminen der Formel R³-NH₂ zu erreichen, wird der Alkohol R²OH vorzugsweise in einer Menge von maximal 100 Moläquivalenten bezogen auf die molare Menge an Hydroxamsäure hinzugefügt.

In **Schema 2** wird ein Reaktionsmechanismus der erfindungsgemäßen Synthese am Beispiel der Verwendung von Dimethylcarbonat (MeO)₂-CO vorgeschlagen. Dieser theoretische Mechanismus zeigt, dass das eingesetzte Amin eine katalytische Funktion bei der Deprotonierung der Hydroxamsäure im Initialschritt hat, und im Folgeschritt wird die Aminbase wieder zurückgebildet. Intermediär wird über eine Lossen-Umlagerung ein Isocyanat gebildet, das aber aufgrund der Anwesenheit von Nukleophilen der Formel Nu-H zum Additionsprodukt abreagiert.

Wird bei dieser Reaktion eine aromatische oder Heteroaromatische Hydroxamsäure eingesetzt, so findet eine Weiterreaktion des Carbamats R³-NH-CO-OR² zum Amin R³-NH₂ statt. Wahrscheinlich ist die für die Umlagerung eingesetzte Base ausreichend, um die Hydrolyse des Carbamats zu bewirken.

Bei der erfindungsgemäßen Synthese ist grundsätzlich überraschend, dass die Lossen-Umlagerung basenkatalytisch unter milden Bedingungen ohne weitere Hilfsstoffe durchgeführt werden kann.

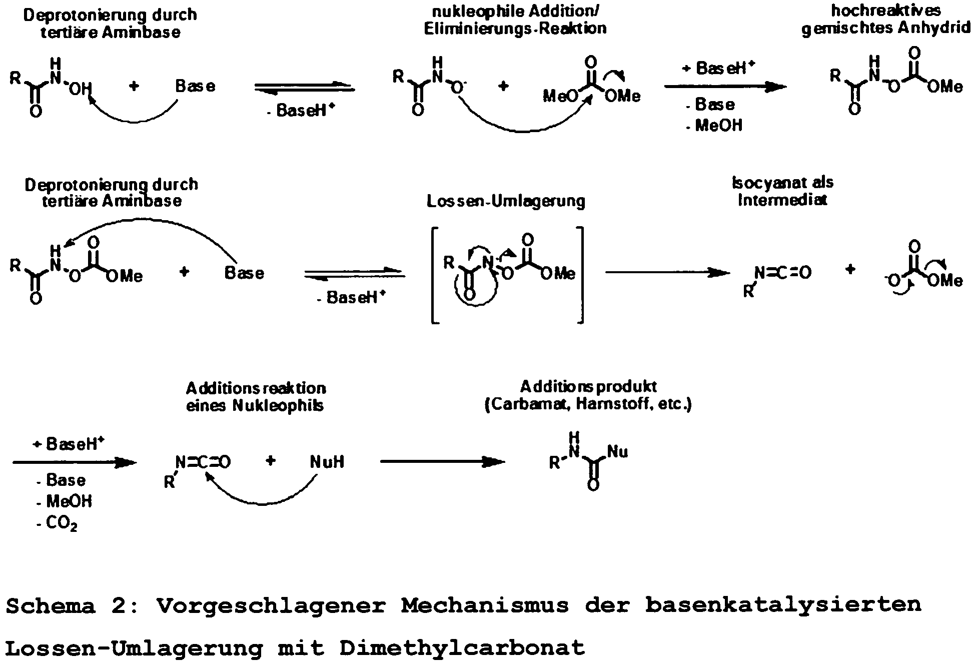

In einer bevorzugten Ausführung des Verfahrens soll bei dieser Synthese ein Carbamat der allgemeinen Formel R¹-NH-CO-OR² hergestellt werden. In einer weiteren bevorzugten Ausführung des Verfahrens sollen Amine der Formel R³-NH₂ hergestellt werden.

Die erfindungsgemäßen Verfahren haben die Vorteile, dass unbedenkliche Reagenzien eingesetzt werden. Die Reagenzien brauchen nicht gereinigt oder getrocknet zu werden, der Einsatz von technischer Qualität ist ausreichend. Es fallen, wie bereits erwähnt, wenig Abfallprodukte an und überschüssige Reagenzien können in einfachen destillativen Schritten rezykliert werden. Lediglich das organische Carbonat wird als einziger Reaktand neben der Hydroxamsäure in stöchiometrischen Mengen umgesetzt, wobei ein Moläquivalent des Alkohols anfällt. Des Weiteren werden die gewünschten Produkte, insbesondere die Carbamte bzw. Amine in guten Ausbeuten isoliert. Die in geringen Umfang isolierten Verunreinigungen (z.B. Carbonsäureester R¹-CO-OR²) lassen sich einfach rezyklieren oder liefern ebenfalls wichtige Zwischenprodukte.

Grundsätzlich besteht die Möglichkeit, dieses Verfahren auch in einem kontinuierlichen Prozess durchzuführen. Da bei dem Verfahren stöchiometrische Mengen an Alkohol R²OH entstehen, wird vorzugsweise bei kontinuierlicher Prozessführung der während der Reaktion entstandene überschüssige Alkohol R²OH abgetrennt.

Diese Erfindung erlaubt es im Gegensatz vieler anderer Verfahren in technisch einfach durchzuführenden Prozessen, mit unbedenklichen Substraten und geringen Anfall von Abfallstoffen eine hochdiverse Produktpalette ausgehend von jeder beliebigen Carbonsäure zu erzeugen.

Die Erfindung wird im Folgenden mit Ausführungsbeispielen erläutert.

### 1. Synthese von Hydroxamsäuren

Nachfolgend wird ein repräsentatives Verfahren für die Synthese von Hydroxamsäuren ausgehend von Methylestern vorgestellt: Die Methylesterverbindungen der Formel R^{1,3}-CO-OMe (40.0 mmol) und Hydroxylammoniumchlorid (8.30 g, 120 mmol, 3.0 eq = Moläquivalente bezogen auf die Methylesterverbindungen) wurden in Methanol (200 mL) suspendiert. Nach Zugabe von Kaliumhydroxid (13.5 g, 240 mmol, 6.0 eq) wurde das Gemisch unter starkem Rühren bis zum Rückfluss erhitzt. Der Reaktionsprozess wurde dünnschichtchromatographisch (DC) überwacht und nach normalerweise eintägiger Reaktion wurde ein vollständiger Umsatz detektiert. Anschließend wurde das Gemisch mit verdünnter Essigsäure (25%) auf pH 4 angesäuert und zur Trockne eingedampft. Der Rückstand wurde in Wasser (50 mL) und Ethylacetat (200 mL) gelöst. Die organische Phase wurde abgetrennt, getrocknet und in Ethylacetat/Hexane Gemisch umkristallisiert. Die reinen Hydroxamsäuren wurden als farblose Feststoffe erhalten.

### 2. Basenkatalysierte Lossen Umlagerungen

Die erfindungsgemäße basenkatalysierte Lossen-Umlagerung wird nach folgendem beispielhaftem Versuchsprotokoll durchgeführt: 100 mmol der Hydroxamsäurederivate werden in 181 g Dimethylcarbonat (entspricht 2 mol bzw. 20 eq = Moläquivalente bezogen auf die eingesetzten Hydroxamsäurederivate) und 6.43 g Methanol (200 mmol bzw. 2 eq) gelöst. Bei der Verwendung anderer Carbonate (Diethylcarbonat, Diallylcarbonat, Diphenylcarbonat, Ethylencarbonat) sowie die entsprechenden Alkohole (Ethanol, Allylalkohol, Phenol, Ethylenglykol) werden die genannten Mengenverhältnisse beibehalten. Das Gemisch wird erhitzt bis zum Einsetzen des Rückfluss und anschließend werden 2.79 g TBD (20 mmol bzw. 0.2 eq) hinzugegeben. Alternativ können auch Triethylamin, DBU,DABCO oder andere tertiäre Amine als Basen eingesetzt werden.

Sobald die Analyse mittels Dünnschichtchromatographie (DC) oder Gaschromatographie mit gekoppelter Massenspektrometrie (GC-MS) eine vollständige Umsetzung anzeigt (typischer Weise nach ca. 20 h) werden die Reaktionsgemische eingedampft. Die Rohprodukte werden danach säulenchromatographisch aufgereinigt, um die reinen Produkte zu erhalten.

### 2.1 Darstellung von Methyldec-9-enylcarbamat

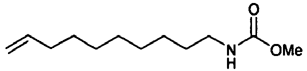

Die Lossen-Umlagerung von N-Hydroxyundec-10-enamide (20.0 g, 100 mmol) mit Dimethylcarbonat (181 g, 2.00 mol, 20 eq), Methanol (6.43 g, 200 mmol, 2 eq) und TBD (2.79 g, 20.0 mmol, 0.2 eq) führte zur Bildung von Methyldec-9-enylcarbamat. Nach der Aufreinigung mittels Säulenchromatographie (n-Hexan/Ethylacetat 9:1 bis 7:3), wurde 12.8 g reines Methyldec-9-enylcarbamat als farbloses Öl erhalten (Ausbeute: 60%).
Analyse: DC (n-Hexan/Ethylacetat 5:1) *R*_{f} = 0.43; ¹H NMR (CDCl₃, 300 MHz) δ = 1.24 - 1.42 (m, 10 H, 5 CH₂), 1.47 (quint., *J* = 6.6 Hz, 2 H, CH₂), 2.03 (q, *J =* 6.8 Hz, 2 H, CH₂), 3.15 (q, *J* = 6.7 Hz, 2 H, CH₂), 3.65 (s, 3 H, CH₃), 4.66 (br, s, 1 H, NH), 4.89 - 5.03 (m, 2 H, CH₂), 5.80 (tdd, *J* = 17.0 Hz, 10.4 Hz, 6.8 Hz, 1 H, CH) ppm; ¹³C NMR (CDCl₃, 75 MHz) δ = 26.65, 28.83, 28.96, 29.15, 29.30, 29.96, 33.70, 41.06, 51.88, 114.09, 139.07, 157.04 ppm; HRMS (hochauflösende Massenspektrometrie) mit FAB (Fast Atomic Bambardment) von C₁₂H₂₃NO₂ [M+H]⁺ theor. 214.1807 gemessen 214.1809; IR (ATR) ν = 3336.5, 3076.2, 2927.1 2854.8, 1702.4 (Carbamat), 1640.3, 1541.1 (Carbamat), 1464.2, 1259.0, 1193.2, 1039.5, 1144.5, 909.3, 779.3 cm⁻¹.

### 2.2 Darstellung von 1,3-Di(dec-9-enyl)harnstoff

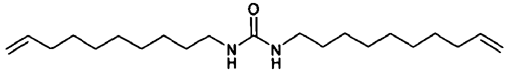

Bei der Aufreinigung von Methyldec-9-enylcarbamat (Bsp. 2.1) kristallisierte 1,3-di(Dec-9-enyl)harnstoff aus. Nach Filtration und Waschen, wurde das Produkt als farblose Kristalle erhalten (4.03 g, 12% Ausbeute).
Analyse: DC (n-Hexan/Ethylacetat 2:1) *R*_{f} = 0.33; ¹H NMR (CDCl₃, 300 MHz) δ = 1.22 - 1.41 (m, 20 H, 10 CH₂), 1.48 (quint., *J* = 6.9 Hz, 4 H, 2 CH₂), 2.03 (q, *J* = 6.7 Hz, 4 H, 2 CH₂, 3.14 (q, *J =* 7.0 Hz, 4 H, 2 CH₂), 4.22 (br, t, *J* = 5.2 Hz, 2 H, 2 NH), 4.89 - 5.03 (m, 4 H, 2 CH₂), 5.80 (tdd, *J =* 17.0 Hz, 10.4 Hz, 6.8 Hz, 2 H, 2 CH) ppm; ¹³C NMR (CDCl₃, 75 MHz) δ = 26.88, 28.88, 29.02, 29.27, 29.37, 30.24, 33.74, 40.64, 114.12, 139.12, 158.33 ppm; HRMS(FAB) von C₂₁H₄₀N₂O [M+H]⁺ theor. 337.3219 gefunden 337.3216; IR (ATR) ν = 3331.7, 2921.3 (Harnstoff), 2849.9 (Harnstoff), 1614.8 (Harnstoff), 1572.3, 1477.5, 1465.6, 1253.7, 1229.5, 1184.7, 990.8, 909.3, 723.2 cm⁻¹.

### 2.3 Darstellung von Methyl(Z)-heptadec-8-enylcarbamat)

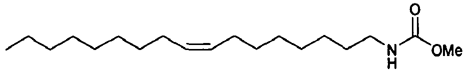

Die Lossen-Umlagerung von N-Hydroxyoleamid (10.0 g, 33.6 mmol) mit Dimethylcarbonat (60.5 g, 672 mol, 20 eq), Methanol (2.15 g, 67.2 mmol, 2 eq) und TBD (0.94 g, 6.72 mmol, 0.2 eq) führte zur Ausbildung von Methyl(Z)-heptadec-8-enylcarbamat. Nach chromatographischer Aufreinigung (n-Hexan/Ethylacetat 5:1 bis 2:1), wurde die Verbindung als farbloses Öl erhalten (6.53 g, Ausbeute: 62%).
Analyse: DC (n-Hexan/Ethylacetat 5:1 bis 2:1) *R*_{f} = 0.44; ¹H NMR (CDCl₃, 300 MHz) δ = 0.87 (t, *J =* 6.8 Hz, 3 H, CH₃), 1.18 - 1.39 (m, 20 H, 10 CH₂), 1.48 (quint., *J =* 6.8 Hz, 2 H, CH₂), 2.00 (q, *J =* 6.7 Hz, 4 H, 2 CH₂), 3.16 (q, *J* = 6.6 Hz, 2 H, CH₂), 3.65 (s, 3 H, CH₃), 4.64 (br, s, 1 H, NH), 5.28 - 5.42 (m, 2 H, 2 CH) ppm; ¹³C NMR (CDCl₃, 75 MHz) δ = 14.04, 22.63, 26.66, 27.11, 27.17, 29.13, 29.27, 29.47, 29.62, 29.71, 29.97, 31.86, 41.06, 51.90, 129.66, 129.97, 157.03 ppm; HRMS(FAB) von C₁₉H₃₇NO₂ [M+H]⁺ theor. 312.2903 gemessen 312.2905; IR (KBr) ν = 3337.8, 3004.3, 2925.7, 2854.6, 1704.8 (Carbamat), 1537.3 (Carbamat), 1464.5, 1378.2, 1258.3, 1193.5, 1144.0, 1036.8, 778.7, 722.8 cm⁻¹.

### 2.4 Darstellung von 1,3-Di((Z)-heptadec-8-enyl)harnstoff

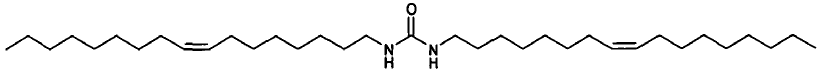

Bei der Aufreinigung von Methyl(Z)-heptadec-8-enylcarbamat (Bsp. 2.3) kristallisiert 1,3-Di((Z)-heptadec-8-enyl)harnstoff aus. Nach Filtration und Waschen, wurde das Produkt als farblose Kristalle erhalten (1.60 g, Ausbeute: 9.0%).
Analyse: DC (n-Hexan/Ethylacetat 2:1) *R*_{f} = 0.51; ¹H NMR (CDCl₃, 300 MHz) δ = 0.88 (t, *J* = 6.8 Hz, 6 H, 2 CH₃), 1.21 - 1.39 (m, 40 H, 20 CH₂), 1.48 (quint., *J* = 6.9 Hz, 4 H, 2 CH₂), 2.00 (q, *J =* 6.7 Hz, 8 H, 4 CH₂), 3.14 (q, *J =* 7.0 Hz, 4 H, 2 CH₂), 4.25 (br, t, *J* = 5.4 Hz, 2 H, 2 NH), 5.27 - 5.42 (m, 4 H, 4 CH) ppm; ¹³C NMR (CDCl₃, 75 MHz) δ = 14.05, 22.62, 26.90, 27.14, 27.18, 29.21, 29.26, 29.28, 29.48, 29.68, 29.72, 30.27, 31.86, 40.36, 129.66, 129.94, 158.53 ppm; HRMS(FAB) von C₃₅H₆₈N₂O [M+H]⁺ calc. 533.5410 gemessen 533.5408; IR (ATR) ν = 3320.3, 2918.1 (Harnstoff), 2848.4 (Harnstoff), 1609.0 (Harnstoff), 1580.1, 1468.4, 1261.2, 1233.4, 728.0, 716.7 cm⁻¹.

### 2.5 Darstellung von Dimethyl-octan-1,8-diyldicarbamat

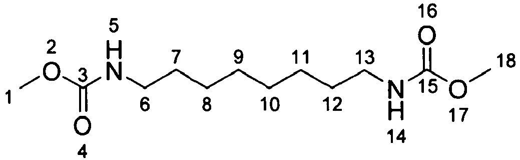

Die Lossen-Umlagerung von *N¹,N¹⁰*-dihydroxydecanediamid (4.42 g, 19.0 mmol) mit Dimethylcarbonate (343 g, 3.81 mol, 200 eq.), methanol (12.2 g, 381 mmol, 20 eq.) und TBD (0.53 g, 3.80 mmol, 0.2 eq) führte zur Ausbildung von Dimethyloctan-1,8-diyldicarbamat. Nach chromatographischer Aufreinigung (Ethylacetat), wurde das Produkt als farbloser Feststoff erhalten (Ausbeute: 1.93 g, 34%).
Analyse: DC (Ethylacetat) *R*_{f} = 0.58; mp = 113°C; ¹H NMR (CDCl₃, 300 MHz) δ = 1.23 - 1.33 (m, 8 H, 4 CH₂*⁸⁻¹¹*), 1.47 (quint., *J* = 6.0 Hz, 4 H, 2 CH₂^{7,12}), 3.15 (q, *J* = 6.5 Hz, 4 H, 2 CH₂*^{6,13}*)*,* 3.65 (s, 6 H, 2 CH₃*^{1,18}*), 4.66 (br, s, 2 H, 2 NH*^{5,14}*) ppm; ¹³C NMR (CDCl₃, 75 MHz) δ = 26.5 (2 CH₂*^{8,11}*), 29.0 (2 CH₂*^{9,10}*), 29.9 (2 CH₂*^{7,12}*), 40.9 (2 CH₂*^{6,13}*), 51.8 (2 CH₃*^{1,18}*), 157.0 (2 CO^{*3,1*5}) ppm; FAB von C₁₂H₂₄N₂O₄ (M+H⁺ = 261.1, M-OMe⁺ = 229.1); HRMS(FAB) von C₁₂H₂₄N₂O₄ [M+H]⁺ calc. 261.1814 gemessen 261.1818; IR (ATR) ν = 3338.8, 2940.1, 2851.7, 1687.2 (Carbamat), 1615.3, 1523.7, 1461.8, 1384.4, 1361.7, 1306.3, 1247.8, 1199.8, 1168.5, 1140.2, 1061.2, 1034.8, 985.2, 938.8, 876.9, 783.2, 756.7, 708.3 cm⁻¹.

### 2.6 Darstellung von Ethyl-dec-9-enylcarbamat

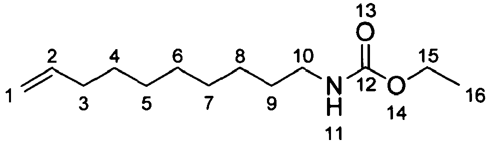

Die Lossen-Umlagerung von *N*-hydroxyundec-10-enamid (1.00 g, 5.02 mmol) mit Diethylcarbonat (11.9 g, 100 mmol, 20 eq), Ethanol (0.46 g, 10.0 mmol, 2.0 eq) und TBD (0.14 g, 1.00 mmol, 0.2 eq) führte zur Ausbildung von Ethyldec-9-enylcarbamat. Nach chromatographischer Aufreinigung (n-Hexan/Ethylacetat 9:1 bis 4:1), wurde das Produkt als farbloses Öl erhalten (Ausbeute: 0.88 g, 77%).
Analyse: DC (n-Hexan/Ethylacetat 5:1) *R*_{f} = 0.47; ¹H NMR (CDCl₃, 300 MHz) δ = 1.23 (t, *J =* 7.0 Hz, 3 H, CH₃*¹⁶*), 1.24 - 1.41 (m, 10 H, 5 CH₂*⁴⁻⁸*), 1.48 (quint., *J =* 6.6 Hz, 2 H, CH₂*⁹*), 2. 03 (q, *J =* 6.8 Hz, 2 H, CH₂*³*), 3.15 (q, *J =* 6.4 Hz, 2 H, CH₂*¹⁰*), 4.10 (q, *J* = 7.0 Hz, 2 H, CH₂*¹⁵*), 4.62 (br, s, 1 H, NH*¹¹*), 4.88-5.03 (m, 2 H, CH₂*¹*), 5.80 (tdd, *J =* 17.0 Hz, 10.4 Hz, 6.6 Hz, 1 H, CH*²*) ppm; ¹³C NMR (CDCl₃, 75 MHz) δ = 14.6 (CH₃*¹⁶*), 26.6 (CH₂*⁸*), 28.8 (CH₂*⁴⁻⁷*), 28.9 (CH₂*⁴⁻⁷*), 29.1 (CH₂*⁴⁻⁷*), 29. 3 (CH₂*⁴⁻⁷*), 29.9 (CH₂*⁹*), 33. 7 (CH₂*³*), 40.9 (CH₂*¹⁰*), 60.5 (CH₂*¹⁵*), 114.1 (CH₂*¹*), 139.0 (CH*²*), 156.6 (CO*¹²*) ppm; FAB of C₁₃H₂₅NO₂ (M+H⁺ = 228.2); HRMS(FAB) von C₁₃H₂₅NO₂ [M+H]⁺ calc. 228.1964 gemessen 228.1965; IR (KBr) ν = 3363.3, 3076.5, 2978.1, 2927.4, 2855.1, 1697.2 (Carbamat), 1640.1, 1535.7, 1461.6, 1380.8, 1253.2, 1140.8, 1042.4, 993.4, 909.0, 778.5 cm⁻¹.

### 2.7 Darstellung von Allyl-dec-9-enylcarbamat

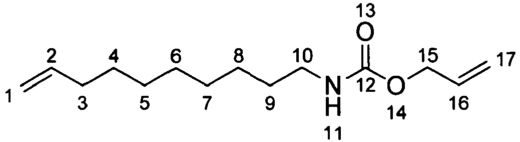

Die Lossen-Umlagerung von *N*-hydroxyundec-10-enamid (1.00 g, 5.02 mmol) mit Diallylcarbonat (14.27 g, 100 mmol, 20 eq), Allylalkohol (0.58 g, 10.0 mmol, 2.0 eq) und TBD (0.14 g, 1.00 mmol, 0.2 eq) führte zur Ausbildung von Allyl-dec-9-enylcarbamat. Nach chromatographischer Aufreinigung (n-Hexan/Ethylacetat 9:1 bis 4:1), wurde das Reinprodukt als farbloses Öl erhalten (Ausbeute: 0.82 g, 68%).
Analytik: DC (n-Hexan/Ethylacetat 5:1) *R*_{f} = 0.53; ¹H NMR (CDCl₃, 300 MHz) δ = 1.22 - 1.42 (m, 10 H, 5 CH₂*⁴⁻⁸*), 1.48 (quint., *J =* 6.6 Hz, 2 H, CH₂*⁹*), 2.03 (q, *J =* 6.8 Hz, 2 H, CH₂*³*), 3.17 (q, *J =* 6.4 Hz, 2 H, CH₂*¹⁰*), 4.55 (d, *J =* 5.3 Hz, 2 H, CH₂*¹⁵*), 4.70 (br, s, 1 H, NH*¹¹*), 4.88 - 5.03 (m, 2 H, CH₂*¹*), 5.16 - 5.34 (m, 2 H, CH₂*¹⁷*), 5.80 (tdd, *J =* 17.0 Hz, 10.2 Hz, 6.8 Hz, 1 H, CH*²*), 5.92 (tdd, *J =* 16.4 Hz, 11.0 Hz, 6.0 Hz, 1 H, CH*¹⁶*) ppm; ¹³C NMR (CDCl₃, 75 MHz) δ = 26.6 (CH₂*⁸*), 28.8 (CH₂^{*4*-7}), 28.9 (CH₂*⁴⁻⁷*), 29.1 (CH₂*⁴⁻⁷*), 29.3 (CH₂*⁴⁻⁷*), 29.9 (CH₂*⁹*), 33.7 (CH₂*³*), 41.0 (CH₂*¹⁰*), 65.3 (CH₂*¹⁵*), 114.1 (CH₂*¹*), 117.4 (CH₂*¹⁷*), 132.9 (CH*¹⁶*), 139.0 (CH*²*), 156.2 (CO*¹²*) ppm; FAB von C₁₄H₂₅NO₂ (M+H⁺ = 240.3); HRMS (FAB) von C₁₄H₂₅NO₂ [M+H]⁺ calc. 240.1964 gemessen 240.1961; IR (KBr) ν = 3345.8, 3078.5, 2927.4, 2855.5, 1703.8 (Carbamat), 1642.7, 1537.3, 1463.6, 1250.6, 1135.9, 993.7, 911.1, 777.4 cm⁻¹.

### 2.8 Darstellung von Phenyl-dec-9-enylcarbamat

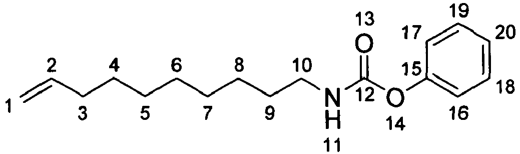

Die Lossen-Umlagerung von *N*-hydroxyundec-10-enamid (1.00 g, 5.02 mmol) mit Diphenylcarbonat (21.5 g, 100 mmol, 20 eq), Phenol (0.94 g, 10.0 mmol, 2.0 eq) und TBD (0.14 g, 1.00 mmol, 0.2 eq) führte zur Ausbildung von Phenyl-dec-9-enylcarbamat. Nach chromatograhpischer Aufreinigung (n-Hexan/Ethylacetat 19:1 bis 5:1), erhielt man das Produkt als farbloses Öl (Ausbeute: 0.86 g, 62%).
Analytik: DC (n-Hexan/Ethylacetat 5:1) *R*_{f} = 0.48; ¹H NMR (CDCl₃, 300 MHz) δ = 1.24 - 1.44 (m, 10 H, 5 CH₂*⁴⁻⁸*), 1.58 (quint., *J =* 6.6 Hz, 2 H, CH₂*⁹*), 2.04 (q, *J = 6.8* Hz, 2 H, CH₂*³*), 3.26 (q, *J* = 6.7 Hz, 2 H, CH₂*¹⁰*), 4.90 - 5.05 (m, 2 H, CH₂*¹*), 5.01 (br, s, 1 H, NH*¹¹*), 5.81 (tdd, *J* = 17.0 Hz, 10.2 Hz, 6.8 Hz, 1 H, CH*²*), 7.12 (d, *J =* 7.6 Hz, 2 H, 2 CH*^{16,17}*), 7.19 (t, *J =* 7.4 Hz, 1 H, CH*²⁰*), 7.35 (t, *J* = 7.7 Hz, 2 H, 2 CH*^{18,19}*) ppm; ¹³C NMR (CDCl₃, 75 MHz) δ = 26.6 (CH₂*⁸*), 28.8 (CH₂*⁴⁻⁷*), 29.0 (CH₂*⁴⁻⁷*), 29.1 (CH₂*⁴⁻⁷*), 29.3 (CH₂*⁴⁻⁷*), 29. 7 (CH₂*⁹*), 33. 7 (CH₂*³*), 41.2 (CH₂*¹⁰*), 114.1 (CH₂*¹*), 121.5 (2 CH*^{16,17}*), 125.1 (CH*²⁰*), 129.1 (2 CH*^{18,19}*)*,* 139.0 (CH*²*), 151.1 (C*¹⁵*), 154.6 (CO*¹²*) ppm; FAB von C₁₇H₂₅NO₂ (M+H⁺ = 276.3); HRMS (FAB) von C₁₇H₂₅NO₂ [M+H]⁺ calc. 276.1964 gemessen 276.1962; IR (KBr) ν = 3332.9, 3073.9, 2926.7, 2854.5, 1716.3 (Carbamat), 1640.0, 1594.9, 1533.5, 1490.7, 1362.4, 1207.6, 1163.3, 1070.4, 1024.7, 993.9, 909.0, 761.7, 688.3 cm⁻¹.

### 2.9 Darstellung von 2-Hydroxyethyl-dec-9-en-1-ylcarbamat

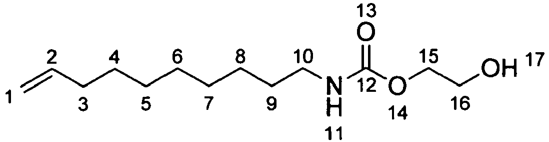

Die Lossen-Umlagerung von *N*-Hydroxyundec-10-enamid (2.00 g, 199 mmol) mit Ethylencarbonat (17.7 g, 201 mmol, 20 eq), Ethylenglycol (1.25 g, 20.1 mmol, 2.0 eq) und TBD (0.28 g, 2.01 mmol, 0.2 eq) führte zur Ausbildung von 2-hydroxyethyl dec-9-en-1-ylcarbamat nach einer Reaktionszeit von 20 Stunden bei 100°C. Das Gemisch wurde anschließend verdünnt mit Ethylacetat (100 mL) und Wasser (30 mL). Nach Abtrennung der wässrige Phase wurde die organische Phase mit Wasser (4 x 30 mL) und mit gesättigter NaCl Lösung (4 x 30 mL) gewaschen. Anschließend wurde die organische Phase über Na₂SO₄ getrocknet, gefiltert und zur Trockne eingedampft. Nach chromatographischer Aufreinigung (n-Hexan/Ethylacetat 7:3 bis reines Ethylacetat), wurde das Produkt als farbloses Wachs erhalten (0.40 g, Ausbeute 19%).
Analytik: DC (*n*-Hexan/Ethylacetat 1:1) *R*_{f} = 0.41; ¹H NMR (CDCl₃, 300 MHz) δ = 1.16 - 1.38 (m, 10 H, 5 CH₂*⁴⁻⁸*), 1.48 (quint., *J =* 6.8 Hz, 2 H, CH₂*⁹*), 1.97 (q, *J=* 6.8 Hz, 2 H, CH₂*³*), 3.06 (br, t, *J =* 6.7 Hz, 1 H, OH*¹⁷*), 3.18 (t, *J =* 7.2 Hz, 2 H, CH₂*¹⁰*), 3.50 (t, *J* = 7. 9 Hz, 2 H, CH₂*¹⁶*), 4.26 (t, *J* = 7. 9 Hz, 2 H, CH₂*¹⁵*), 4.82 - 5.02 (m, 2 H, CH₂*¹*), 5.74 (tdd, *J* = 17.0 Hz, 10.2 Hz, 6.8 Hz, 1 H, CH*²*) ppm; ¹³C NMR (CDCl₃, 75 MHz) δ = 26.4 (CH₂*⁹*), 27.1 (CH₂*⁴⁻⁸*), 28.7 (CH₂*⁴⁻⁸*), 28.8 (CH₂*⁴⁻⁸*), 29.0 (CH₂*⁴⁻⁸*), 29.1 (CH₂*⁴⁻⁸*), 30.1 (CH₂*⁴⁻⁸*), 33.5 (CH2*³*), 40.2 (CH₂*¹⁰*), 44.0 (CH₂*¹⁶*), 61.5 (CH₂*¹⁵*), 114.0 (CH₂*¹*), 138.9 (CH*²*), 158.3 (CO*¹²*) ppm; FAB of C₁₃H₂₅NO₃ (M+H⁺ = 244.4, M-OH⁺ = 226.4); HRMS(FAB) von C₁₃H₂₅NO₃ [M+H]⁺ calc. 244.1913 gemessen 244.1911; IR (ATR) ν = 3329.4, 3251.8, 3076.6, 2922.7, 2851.1, 1807.8, 1734.1 (Carbamat), 1640.0, 1615.1, 1571.1, 1484.3, 1428.0, 1372.6, 1257.8, 1151.3, 1071.8, 993.2, 970.9, 908.4, 762.6, 721.3 cm⁻¹.

### 2.10 Darstellung von Methyl-4-hydroxy-3-methoxyphenethyl-carbamat

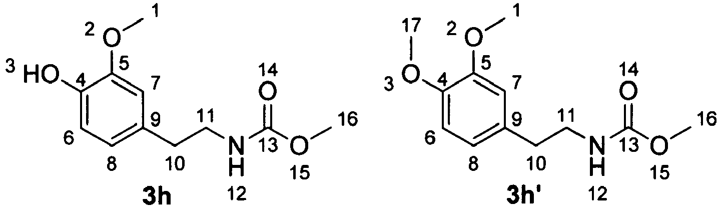

Die Lossen-Umlagerung von *N*-hydroxy-3-(4-hydroxy-3-methoxyphenyl)propanamid (2.24 g, 10.6 mmol) mit Dimethylcarbonat (19.1 g, 212 mmol, 20 eq), Methanol (0.68 g, 21.2 mmol, 2.0 eq) und TBD (0.59 g, 4.24 mmol, 0.4 eq) führte zur Ausbildung von Methyl 4-hydroxy-3-methoxyphenethylcarbamat. Die Lösung wurde zur Trockne eingedampft und das Rohprodukt wurde mit 1 N HCl (50 mL) und Ethylacetat (50 mL) versetzt. Die organische Phase wurde abgetrennt und über Na₂SO₄ getrocknet. Nach chromatographischer Aufreinigung (n-Hexan/Ethylacetat 2:1 bis reines Ethylacetat), wurde ein Gemisch von 3h und 3h' als hellgelbes Öl erhalten (ratio ∼1:1, Ausbeute: 1.81 g, 76%).
Analytik: DC (*n*-Hexan/Ethylacetat 1:1) *R*_{f} = 0.33; ¹H NMR (CDCl₃, 300 MHz, **3h** und **3h'** ratio ∼1:1) δ = 2.74 (q, *J* = 6.6 Hz, 2 H, CH₂*¹⁰*), 3.41 (quint., *J* = 5.5 Hz, 2 H, CH₂*¹¹*), 3.66 (s, 3 H, CH₃*¹⁶*), 3.86, 3.87 (2s, 6 H, CH₃*^{1,17}*), 4.68 (br, s, 1 H, NH*¹²*), 5.53 (br, s, 1 H, OH*³*), 6. 64 - 6.75 (m, 2 H, 2 CH*^{7,8}*), 6.83 (2d, *J* = 8.2 Hz, 1 H, CH*⁶*) ppm; ¹³C NMR (CDCl₃, 75 MHz, **3h** und **3h*'*** ratio ∼1:1) δ = 35.6 (CH₂*¹⁰*), 35.6 (CH₂*¹⁰*), 42.2 (CH₂*¹¹*), 42.3 (CH₂*¹¹*), 51.9 (CH₃*¹⁶*), 55.7 (CH₃*^{1,17}*), 55.7 (CH₃*^{1,17}*), 111.2 (CH*⁷*), 111.3 (CH*⁷*), 111.8 (CH*^{6,3h'}*), 114.4 (CH^{*6*,3h}), 120.6 (CH*⁸*), 121.2 (CH*⁸*), 130.4 (C*⁹*), 131.2 (C*⁹*), 144.2 (C^{*4*,3h}), 146.6 (C^{*4*,3h'}), 147.6 (C*⁵*), 148.9 (C*⁵*), 157.0 (C*¹³*) ppm; FAB of C₁₁H₁₅NO₄ **(3h,** M⁺= 225.0); FAB von C₁₂H₁₇NO₄ **(3h',** M+H⁺ = 240.1, M+ = 239.1); HRMS (FAB) von C₁₁H₁₅NO₄ **3h** M⁺ calc. 225.1001 gemessen 225.0098; HRMS (FAB) von C₁₂H₁₇NO₄ **3h'** M⁺ calc. 239.1158 gemessen 239.1156; IR (KBr) ν = 3359.1, 2942.2, 2838.2, 1703.8 (Carbamat), 1602.1, 1516.2, 1464.2, 1368.5, 1264.5, 1236.8, 1195.1, 1154.8, 1062.3, 1029.9, 936.0, 852.1, 812.1, 779.0 cm⁻¹.

### 2.11 Darstellung von Anilin

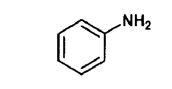

Die Lossen-Umlagerung von N-Hydroxybenzamid (1.18 g, 8.60 mmol) mit Dimethylcarbonat (15.5 g, 172 mol, 20 eq), Methanol (0.55 g, 17.2 mmol, 2 eq) und TBD (0.24 g, 1.72 mmol, 0.2 eq) führte direkt zur Bildung von Anilin, ohne dass das erwartete Methylcarbamat als Zwischenprodukt isoliert werden konnte. Nach säulenchromatographischer Aufreinigung (*n*-Hexan/Ethylacetat 5:1 bis 2:1), wurde reines Anilin als hellgelbe Flüssigkeit erhalten (0.65 g, Ausbeute: 81%).
Analyse: DC (n-Hexan/Ethylacetat 2:1) *R*_{f} = 0.40; ¹H NMR (CDCl₃, 300 MHz) δ = 3.71 (br, s, 2 H, NH₂), 6.69 - 6.74 (m, 2 H, 2 CH), 6.78 (tt, *J =* 7.4 Hz, 1.1 Hz, 1 H, CH), 7.12 - 7.22 (m, 2 H, 2 CH) ppm; ¹³C NMR (CDCl₃, 75 MHz) δ = 115.07, 118.52, 129.22, 146.25 ppm.

### 2.12 Darstellung von 3,4,5-Trimethoxyanilin

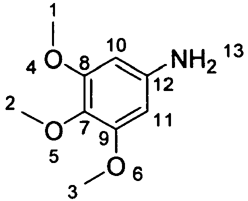

Die Lossen-Umlagerung von *N*-hydroxy-3,4,5-trimethoxybenzamid (1.00 g, 4.40 mmol) mit Dimethylcarbonat (7.93 g, 88.0 mmol, 20 eq), Methanol (0.28 g, 8.80 mmol, 2.0 eq) und TBD (0.12 g, 0.88 mmol, 0.2 eq) führte zur Ausbildung von 3,4,5-trimethoxyanilin nach dreistündigem Erhitzen unter Rückfluss. Nach chromatographischer Aufreinigung(*n*-Hexan/Ethylacetat 4:1 bis 1:2), wurde das reine Produkt als farblose Kristalle erhalten (Ausbeute: 0.65 g, 81%).
Analytik: DC (n-Hexan/Ethylacetat 1:1) *R*_{f} = 0.21; ¹H NMR (CDCl₃, 300 MHz) δ = 3.56 (br, s, 2 H, NH₂*¹³*), 3.76 (s, 3 H, CH₃*²*), 3.80 (s, 6 H, 2 CH₃*^{1,3}*), 5.93 (s, 2 H, 2 CH*^{10,11}*) ppm; ¹³C NMR (CDCl₃, 75 MHz) δ = 55.8 (2 CH₃*^{1,3}*), 60.9 (CH₃*²*), 92.5 (2 CH^{*10*,*11*})*,* 130.6 (C*⁷*), 142.9 (C*¹²*), 153.7 (2 C*^{8,9}*) ppm.

### 2.13 Darstellung von o-Bromoanilin

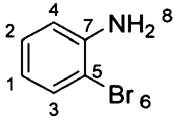

Die Lossen-Umlagerung von o-Bromo-N-hydroxybenzamid (1.00 g, 4.63 mmol) mit Dimethylcarbonat (8.34 g, 92.6 mmol, 20 eq), Methanol (0.30 g, 9.26 mmol, 2.0 eq) und TBD (0.13 g, 0.93 mmol, 0.2 eq) führte zur Ausbildung von o-Bromoanilin. Nach chromatographischer Aufreinigung (n-Hexan/Ethylacetat 9:1 bis 2:1), wurde das Produkt als farbloser Feststoff erhalten (Ausbeute: 0.58 g, 73%).
Analytik: DC (Ethylacetat) *R*_{f} = 0.79; ¹H NMR (CDCl₃ 300 MHz) δ = 4.07 (br, s, 2 H, NH₂*⁸*), 6.59 - 6.66 (m, 1 H, CH*⁴*), 6.77 (dd, *J =* 8.1 Hz, 1.5 Hz, 1 H, CH*¹*), 7.06 - 7.15 (m, 1 H, CH*²*), 7.41 (dd, *J* = 8.1 Hz, 1.3 Hz, 1 H, CH*³*) ppm; ¹³C NMR (CDCl₃, 75 MHz) δ = 109.3 (C*⁵*), 115.7 (CH*⁴*), 119.4 (CH*¹*), 128.3 (CH*²*), 132.5 (CH*³*), 144.0 (C⁷) ppm.

### 2.14 Darstellung von p-Toluidin

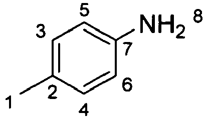

Die Lossen-Umlagerung von *N*-hydroxy-*p*-methylbenzamid (1.00 g, 5.98 mmol) mit Dimethylcarbonat (10.8 g, 120 mmol, 20 eq), Methanol (0.38 g, 12.0 mmol, 2.0 eq) und TBD (0.17 g, 1.20 mmol, 0.2 eq) führte zur Ausbildung von *p*-toluidine Nach chromatographischer Aufreinigung (*n*-Hexan/Ethylacetat 4:1 bis 2:1), wurde das Produkt als hellgelber Feststoff erhalten (Ausbeute: 0.55 g, 78%).
Analytik: DC (*n*-Hexan/Ethylacetat 1: 1) *R_{f}* = 0.69; ¹H NMR (CDCl₃, 300 MHz) δ = 2.25 (s, 3 H, CH₃*¹*), 3.57 (br, s, 2 H, NH₂*⁸*), 6.62 (d, *J* = 8.3 Hz, 2 H, 2 CH*^{5,6}*), 6.98 (d, *J =* 7.9 Hz, 2 H, 2 CH*^{3,4}*) ppm; ¹³C NMR (CDCl₃, 75 MHz) δ = 20.4 (CH₃*¹*), 115.2 (2 CH*^{5,6}*), 127.7 (CH*²*), 129.7 (2 CH*^{3,4}*), 143.8 (CH*⁷*) ppm.

### 2.15 Darstellung von p-Nitroanilin

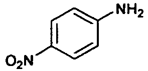

Die Lossen-Umlagerung von N-hydroxy-*p*-nitrobenzamid (0.50 g, 2.75 mmol) mit Dimethylcarbonat (4.95 g, 54.9 mol, 20 eq) und TBD (0.02 g, 0.14 mmol, 0.05 eq) wurde in Abwesenheit von Methanol durchgeführt. Auch hier führte die Reaktion direkt zur Bildung des Amins (*p*-Nitroanilin). Die Nitrogruppe in para-Stellung wurde nicht angegriffen. Nach zwanzigstündiger Reaktionszeit unter Rückfluss und Aufreinigung über Säulenchromatographie (*n*-Hexan/Ethylacetat 4:1 bis 1:1), wurde reines *p-*Nitroanilin als gelber Feststoff erhalten. (0.27 g, Ausbeute: 72%).
Analyse: DC (*n*-Hexan/Ethylacetat 1:1) *R*_{f} = 0.55; ¹H NMR (CDCl₃, 300 MHz) δ = 4.39 (br, s, 2 H, NH₂), 6.63 (d, *J* = 8.9 Hz, 2 H, 2 CH), 8.07 (d, *J =* 8.9 Hz, 2 H, 2 CH) ppm.

## Patentansprüche

1. Verfahren zur Herstellung von Carbamaten oder Harnstoffen der allgemeinen Formel R¹-NH-CO-Nu oder von aromatischen Aminen der allgemeinen Formel R³-NH₂, wobei
Nu ein Alkoxyrest der Formel OR² oder ein Alkylamin mit einer Formel NHR¹ ist, und wobei
R¹ ein ggf. substituierter, linearer, verzweigter oder cyclischer C₁-C₅₀ Alkyl-, Alkenyl- oder Allylrest ist,
R² ein ggf. substituierter, linearer, verzweigter oder cyclischer C₁-C₅₀ Alkyl-, Alkenyl- oder Allylrest, oder ein C₆-C₅₀ Aryl- oder C₄-C₅₀ Heteroarylrest ist, und wobei
R³ ein C₆-C₅₀ Arylrest oder C₄-C₅₀ Heteroarylrest ist, umfassend folgende Verfahrensschritte:
i. Bereitstellen einer Hydroxamsäure der allgemeinen Formel R¹-CO-NH-OH oder R³-CO-NH-OH und
ii. Umsetzen der Hydroxamsäure mit organischen Carbonaten der Formel R²O-CO-OR² in Gegenwart eines tertiären Amins.

2. Verfahren nach Anspruch 1, wobei Schritt ii) in Gegenwart eines Alkohols der Formel R²OH durchgeführt wird, wobei R² definiert ist wie oben.

3. Verfahren nach Anspruch 2, wobei der Alkohol in einer Menge von maximal 100 Moläquivalenten bezogen auf die molare Menge an Hydroxamsäure hinzugefügt wird.

4. Verfahren nach Anspruch 1 bis 3, wobei die Umsetzung in Schritt ii) mit organischen Carbonaten in einer Menge von 0,1 bis 500 Moläquivalenten bezogen auf die molare Menge an Hydroxamsäure erfolgt.

5. Verfahren nach Anspruch 1 bis 4, wobei die Umsetzung in Schritt ii) mit einem tertiären Amin in einer Menge von 0,0001 bis 2 Moläquivalenten bezogen auf die molare Menge an Hydroxamsäure erfolgt.

6. Verfahren nach Anspruch 1 bis 5, wobei das Verfahren bei kontinuierlicher Prozessführung durchgeführt wird und der während der Reaktion entstandene Alkohol R²OH abgetrennt wird.

7. Verfahren nach einem der Ansprüche 1 bis 6, wobei das tertiäre Amin aus der Gruppe 1,5,7-Triazabicyclo[4.4.0]dec-5-en (TBD), 1,8-Diazabicyclo[5.4.0]undec-7-en (DBU) und 1,4-Diazabicyclo[2.2.2]octan (DABCO) ausgewählt ist.

8. Verfahren nach einem der Ansprüche 1 bis 7, wobei R² eine Methylgruppe ist.

9. Verfahren nach Anspruch 1 bis 8, wobei ein Carbamat der allgemeinen Formel R¹-NH-CO-OR² hergestellt wird.

10. Verfahren nach Anspruch 1 bis 8, wobei ein Amin der allgemeinen Formel R³-NH₂ hergestellt wird.

## Claims

1. Method for the production of carbamates or ureas of the general formula R¹-NH-CO-Nu, or of aromatic amines of the general formula R³-NH₂, wherein
Nu is an alkoxy residue of the formula OR² or an alkyl amine with a formula NHR¹, and wherein
R¹ is, as applicable, a substituted, linear, branched, or cyclic C₁-C₅₀ alkyl residue, alkenyl residue, or allyl residue,
R² is, as applicable, a substituted, linear, branched, or cyclic C₁-C₅₀ alkyl residue, alkenyl residue, or allyl residue, or a C₆-C₅₀ aryl residue or C₄-C₅₀ heteroaryl residue, comprising the following method steps:
i. provision of a hydroxamic acid of the general formula R¹-CO-NH-OH or R³-CO-NH-OH and
ii. reacting of the hydroxamic acid with organic carbonates of the formula R²O-CO-OR² in the presence of a tertiary amine.

2. Method according to claim 1, wherein step ii) is carried out in the presence of an alcohol of the formula R²OH, where R² is defined as above.

3. Method according to claim 2, wherein the alcohol is added in a quantity of maximum 100 mol equivalents related to the molar quantity of hydroxamic acid.

4. Method according to claim 1, wherein the reaction in step ii) takes place with organic carbonates in a quantity of 0.1 to 500 mol equivalents related to the molar quantity of hydroxamic acid.

5. Method according to claims 1 to 4, wherein the reaction in step ii) takes place with a tertiary amine in a quantity of 0.0001 to 2 mol equivalents related to the molar quantity of hydroxamic acid.

6. Method according to claims 1 to 5, wherein the method is carried out with continuous performance of the process, and the alcohol R²OH incurred during the reaction is separated off.

7. Method according to any one of claims 1 to 6, wherein the tertiary amine is selected from the group 1,5,7-triazabicyclo[4.4.0]dec-5-ene (TBD), 1,8-diazabicyclo]5.4.0]undec-7-ene (DBU) and 1,4-diazabicyclo[2.2.2]octane (DABCO).

8. Method according to any one of claims 1 to 7, wherein R² is a methyl group.

9. Method according to any one of claims 1 to 8, wherein a carbamate of the general formula R¹-NH-CO-OR² is produced.

10. Method according to any one of claims 1 to 8, wherein an amine of the general formula R³-NH₂ is produced.

## Revendications

1. Procédé de préparation de carbamates ou de carbamides, de formule générale R¹-NH-CO-Nu ou d'amines aromatiques de formule générale R³-NH₂ dans lesquelles :
Nu est un résidu alkoxy de formule OR² ou une alkylamine de formule NHR¹, et
R¹ représente un résidu alkyl, un résidu alcényl ou un résidu allyl, en C₁-C₅₀ linéaire, ramifié ou cyclique le cas échéant substitué,
R² représente un résidu alkyl, alcényl ou allyl en C₁-C₅₀ linéaire, ramifié ou cyclique le cas échéant substitué, ou un résidu aryl en C₆-C₅₀ ou un résidu hétéroaryl en C₄-C₅₀ et
R3 représente un résidu aryl en C₆-C₅₀ ou un résidu hétéroaryl en C₄-C₅₀,
comprenant les étapes suivantes consistant à :
i. préparer un acide hydroxamique de formule générale R¹-CO-NH-OH ou R³-CO-NH-OH, et
ii. faire réagir l'acide hydroxamique avec des carbonates organiques de formule R²O-CO-OR² en présence d'une amine tertiaire.

2. Procédé conforme à la revendication 1, selon lequel l'étape ii) est mise en oeuvre en présence d'un alcool de formule R²OH, R² étant défini comme ci-dessus.

3. Procédé conforme à la revendication 2, selon lequel l'alcool est ajouté en quantité d'au maximum 100 équivalents molaires par rapport à la quantité molaire d'acide hydroxamique.

4. Procédé conforme à l'une des réactions 1 à 3 selon lequel la réaction dans l'étape ii) est mise en oeuvre avec des carbonates organiques en quantité de 0,1 à 500 équivalents molaires par rapport à la quantité molaire d'acide hydroxamique.

5. Procédé conforme à l'une des revendications 1 à 4, selon lequel la réaction dans l'étape ii) est mise en oeuvre avec une amine tertiaire en quantité de 0,0001 à 2 équivalents molaires par rapport à la quantité molaire d'acide hydroxamique.

6. Procédé conforme à l'une des revendications 1 à 5, selon lequel le procédé est mis en oeuvre en continu et l'alcool R²OH formé au cours de la réaction est extrait.

7. Procédé conforme à l'une des revendications 1 à 6, selon lequel l'amine tertiaire est choisie dans le groupe des amines tertiaires suivantes :1, 5, 7-Triazabicyclo [4.4.0] dec-5-éne (TBD), 1,8-Diazabicyclo [5.4.0]undec-7-éne (DBU) et 1,4-Diazabicyclo [2.2.2]octane (DABCO).

8. Procédé conforme à l'une des revendications 1 à 7, selon lequel R² représente un groupe méthyl.

9. Procédé conforme à l'une des revendications 1 à 8, selon lequel on prépare un carbamate de formule générale R¹-NH-CO-OR².

10. Procédé conforme à l'une des revendications 1 à 8, selon lequel on prépare une amine de formule générale R³-NH₂.
